# EUROPEAN PATENT SPECIFICATION

(11) **EP 1 331 948 B1**
(45) Date of publication and mention of the grant of the patent: **13.09.2006**
(21) Application number: 01982929.0
(22) Date of filing: 12.10.2001
(51) Int. Cl.: A61K 47/44, A61K 35/64

(54) **PHARMACEUTICAL COMPOSITION CONTAINING HONEY FOR THE TREATMENT OF WOUNDS**
HONIG ENTHALTENDE PHARMAZEUTISCHE ZUSAMMENSETZUNG ZUR WUNDBEHANDLUNG
COMPOSITION COMPRENANT DU MIEL DESTINEE A SOIGNER DES PLAIES

(30) Priority: 13.10.2000 NL 1016398
(43) Date of publication of application: 06.08.2003
(73) Proprietor: Triticum Exploitatie B.V., 6211 TE Maastricht (NL)
(72) Inventor: POSTMES, Theodore, Justin, Louis, Joseph, NL-6211 TE Maastricht (NL)
(74) Representative: Valkonet, Rutger
(86) International application number: PCT/NL2001/000754
(87) International publication number: WO 2002/030467

(56) References cited:
- EP-A- 0 692 254
- FR-A- 2 773 711
- US-A- 5 980 875
- J. VANDEPUTTE: "Bee's best skin care products - Scientific information" INTERNET ARTICLE, [Online] August 2000 (2000-08), pages 1-9, XP002169765 Retrieved from the Internet: <URL:http://www.triticum.nl/english/s_arti cles/skin_care.htm> [retrieved on 2001-06-13]
- KERKVLIET J D: "Screening method for the determination of peroxide accumulation in honey and relation with HMF content." JOURNAL OF APICULTURAL RESEARCH, vol. 35, no. 3-4, 1996, pages 110-117, XP001002866 ISSN: 0021-8839
- ANONYMOUS: "Scientific articles on the medicinal use of honey" INTERNET ARTICLE, [Online] XP002169766 Retrieved from the Internet: <URL:http://www.triticum.nl/english/s_arti cles/sart_coe.html> [retrieved on 2001-06-13]
- POSTMES THEO ET AL: "Honey for wounds, ulcers, and skin graft preservation." LANCET (NORTH AMERICAN EDITION), vol. 341, no. 8847, 1993, pages 756-757, XP002169767 ISSN: 0099-5355
- MOLAN P C: "The antibacterial activity of honey 1. The nature of the antibacterial activity" BEE WORLD, BEE RESEARCH ASSOCIATION, GERRARDS CROSS, GB, vol. 1-2, 1992, pages 5-29, XP002902515 ISSN: 0005-772X
- POSTMES T ET AL: "The sterilization of honey with cobalt 60 gamma radiation: A study of honey spiked with spores of Clostridium botulinum and Bacillus subtilis." EXPERIENTIA (BASEL), vol. 51, no. 9-10, 1995, pages 986-989, XP002169769 ISSN: 0014-4754
- DATABASE WPI Section Ch, Week 199846 Derwent Publications Ltd., London, GB; Class B04, AN 1998-540603 XP002169770 & RU 2 107 510 C (NIKITIN P G), 27 March 1998 (1998-03-27)
- DATABASE WPI Section Ch, Week 200025 Derwent Publications Ltd., London, GB; Class B04, AN 2000-291326 XP002169771 & RU 2 127 584 C (KONOVALOV V N), 20 March 1999 (1999-03-20)

## Description

The present invention relates to a composition comprising honey as a therapeutically active compound, for the treatment of wounds, which composition furthermore comprises lanolin and/or a lanolin derivative. More in particular, the present invention relates to the use of a honey-containing composition for the preparation of a substance for the treatment of wounds.

Such a composition is known per se from US patent no. 5,785,972. The composition that is known therefrom comprises a therapeutically active compound with antiseptic, osmotic and further characteristics, which composition is used in particular for the treatment of burns and open wounds experienced by animals and man and in particular for the treatment of thermal burns on humans by use of a spray composition. This composition comprises a combination of colloidal silver, raw honey and helichrysum oil, emulsified with an emulsifying agent so as to form a solution. Water soluble lecithin is mentioned as a suitable emulsifying agent. Raw honey is believed to provide antiseptic qualities and to retard the loss of fluids from the trauma site. Additional information with regard to the honey that is used therein is not provided, whilst moreover the emphasis is laid on the antibacterial activity of the emulsion that is used. Honey based substances for the treatment of wounds are furthermore known from European patent application no. 0 692 254, French patent application no. 2 773 711 and US patent no. 5,980,875. Further information with regard to the special honey cannot be derived therefrom, however.

The object of the present invention is to provide a composition with a base of honey as a therapeutically active compound, which composition is suitable for the treatment of wounds, such as first-degree and second-degree burns, open wounds (chronic wounds), diabetic foot wounds, bedsores (decubitus) and other skin wounds.

According to the present invention, the composition as referred to in the introduction is characterized in that the honey has a peroxide number > 5 µg/g honey.hour, measured at 21 °C.

The present combination of honey having the present peroxide number, lanolin and/or a lanolin derivative has appeared to be suitable for the treatment of chronic wounds, whereby in particular low-allergen type lanolin is sued. If honey having a peroxide count outside the aforesaid range is used, the desired antibacterial activity of the honey will be insufficient. A honey having a peroxide number > 5 µg/g honey.hour, measured at 21 °C, helps stimulate the cell division (mitosis) both of the cells in the dermis and of the cells in the epidermis.

The term "lanolin and/or lanolin derivative" as used in the present description includes, besides lanolin, a complex mixture of esters, diesters and hydroxy esters of high molecular weight lanolin alcohols and high molecular weight lanolin acids. Lanolin, which can be considered as a by-product of the wool industry, is an agent which exhibits a strong softening activity, which, by subjective evaluation, has a softening or improving effect on dry or rough skin caused by the absence of an adequate natural moisture retention. Lanolin thus helps maintain or restore the required skin moisture content.

Preferably, the present composition furthermore comprises zinc oxide, in particular zinc oxide having a particle size < 0.1 µm. The use of zinc oxide has a positive effect on the wound healing process, in particular as a result of the production of an insulin-like growth factor.

Preferably, the present invention furthermore comprises one or more additional components selected from the group of antioxidants, fully trans retinoinic acid (vitamin A acid) or derivatives therefrom, polyunsaturated fatty acids, n-hexacosanol, bis(maltolato) oxovanadium(IV), aloë vera, calendula oil, ascorbyl palmitate, ascorbic acid, vitamin E, thickener, such as carboxymethyl cellulose, polyethylene glycol and starch, which may or may not be modified.

Especially preferred is vitamin A or a derivative therefrom, for example a vitamin A-containing composition such as cod-liver oil. Vitamin A can be considered to be a precursor of vitamin A acid and is conducive to the healing of wounds. The addition thereof is in particular desirable if a patient's healing process is retarded, for example due to the administration of corticosteroids. It is assumed that vitamin A or a derivative therefrom stimulates the production of type I and type II collagen.

In specific embodiments it is furthermore desirable to add propolis, a bee product which is known to those skilled in the art, to the present substance, in particular for the treatment of radiation wounds. Calendula oil is in particular preferred because it is believed to have a therapeutic effect. In addition to this, it is desirable to use a compound having both hydrophilic and lipophilic properties, such as ascorbyl palmitate.

The honey which is used in the present composition is in particular preferred to possess one or more special properties.

As described above, it is in particular desirable for the honey to have a peroxide number > 5 µg/g honey.hour, measured at 21 °C.

If honey having a peroxide number outside the aforesaid range is used, the desired antibacterial activity of the honey will be insufficient. If a cosmetic skin ointment is desired, for which the antibacterial activity is not critical, it may be desirable in certain embodiments to add simple sugars (glucose and fructose, saccharose) to the honey.

Since the present composition is directly applied to the wound, the honey that is used is in particular preferred to be sterile. Sterile honey can be obtained by subjecting it to a sterilization treatment, in particular sterilization by means of gamma rays, for example gamma rays from cobalt 60. Experiments have shown that the honey retains its antibacterial activity after such radiation. The special requirement that the honey used in the composition be sterile obtains in particular with regard to a number of uses, for example in the treatment of otitis extra, inflammation in the nose, or in the treatment of a breastfeeding mother's cracked nipples. It should be understood, however, that such a sterilisation treatment is to be considered optional.

In a special embodiment, the honey that is used in the present invention is in particular preferred to be free from heavy metals, pesticides and herbicides. This is in particular desirable because the present composition is directly applied to the skin wound.

The amount of honey that is used in the present composition ranges from 5-95 wt. %, in particular from 10-90 wt. %, based on the weight of the overall composition.

The amount of lanolin and/or lanolin derivative that is used in the present composition ranges from 5-95 wt. %, in particular from 10-90 wt. %, based on the weight of the overall composition.

The amount of zinc oxide that is used in the present composition ranges from 0.5-10 wt. %, in particular from 1.5-5 wt. %, based on the weight of the overall composition.

The amount of the aforesaid additional components in the present composition preferably ranges from 10-50 wt. %, based on the weight of the overall composition.

If the amount of honey does not fall within the aforesaid range, the special activity of the present composition for the treatment of wounds will not be observed to a sufficient degree. Inadequate therapeutic activity will also be observed if the amount of lanolin and/or lanolin derivative does not fall within the aforesaid range. The same obtains with regard to the amount of zinc oxide and/or additional components that are used in the present composition.

The present composition can be used both in the form of a liquid solution and in the form of an ointment.

The present invention will now be explained by means of a number of examples.

### Comparative example 1.

Pure honey having a peroxide count of 0.0 µg/g honey.hour, measured at 21 °C, was applied to the wound site of a person having serious decubitus. Although a slight improvement in the healing process of the bedsores was achieved in comparison with the situation wherein no substance was applied, the patient experienced undesirable pain.

### Comparative example 2.

A composition consisting of 10 wt. % of lanolin and 90 wt.% of honey having a peroxide count of 3 µg/g honey.hour, measured at 21 °C, was used with a group of patients having serious decubitus. The healing of the wounds was slightly accelerated, albeit to an insufficient degree.

### Example 1.

A composition consisting of 10 wt. % of lanolin and 90 wt.% of honey was used with a group of patients having serious decubitus.. The honey had a peroxide count of 25 µg/g honey.hour, measured at 21 °C. The bedsores healed remarkably fast, a reduction upwards of 20% was observed, whereby in addition the pain that was experienced in comparative example 1 was fully gone.

### Example 2.

The composition of example 1 was used, with this difference that additional 3 wt. % of zinc oxide was used. The healing rate was comparable to that of example 1, with this difference that the stimulation of the production of an insulin-like growth factor slightly accelerated the healing of the wounds.

### Example 3.

The composition of example 2 was used, with this difference that additional 15 wt. % of cod-liver oil was used. A group of patients having open wounds who were treated with this composition exhibited a very fast healing process, whereby in addition the skin fully recovered its smooth surface structure.

### Comparative example 3.

A composition consisting of 95 wt. % of lanolin and 5 wt. % of zinc oxide was used with a group of patients having deep grazes. Although this composition, which had a peroxide count of 0.0 µg/g honey.hour, measured at 21 °C, exhibited some therapeutic activity, the time it took for the wounds to heal was considerably longer than in the situation wherein a composition was used which contained honey in addition to lanolin.

### Example 4.

A composition consisting of 50 wt. % of lanolin and 50 wt.% of honey having a peroxide count of 8 µg/g honey.hour, measured at 21°C, was used with a group of patients having deep grazes. The composition was experienced as pleasant by the patients, and the wounds were healed within two weeks.

## Claims

1. A composition comprising honey as a therapeutically active compound for the treatment of wounds, which composition furthermore comprises lanolin and/or a lanolin derivative, **characterized in that** said honey has a peroxide number > 5 µg/g honey.hour, measured at 21°C.

2. A composition according to claim 1, **characterized in that** low-allergen type lanolin is used.

3. A composition according to claims 1 - 2, **characterized in that** said composition furthermore comprises zinc oxide.

4. A composition according to claims 3, **characterized in that** zinc oxide having a particle size < 0.1 µm is used.

5. A composition according to claims 1 - 4, **characterized in that** said composition comprises one or more additional components selected from the group of antioxidants, fully trans vitamin A acid, polyunsaturated fatty acids, n-hexacosanol, bis(maltolato) oxovanadium(IV), aloë vera, calendula oil, ascorbyl palmitate, ascorbic acid, vitamin E, thickener, such as carboxymethyl cellulose, polyethylene glycol and starch, which may or may not be modified.

6. A composition according to claims 1- 5, **characterized in that** said honey has been subjected to a sterilization treatment, in particular sterilization by means of gamma rays

7. A composition according to claims 1 - 6, **characterized in that** said honey is free from heavy metals, pesticides and herbicides.

8. A composition according to claims 1 - 7, **characterized in that** the amount of honey ranges from 5-95 wt. %, based on the weight of the overall composition.

9. A composition according to claims 1 - 7, **characterized in that** the amount of lanolin and/or lanolin derivative ranges from 5-95 wt.%, based on the weight of the overall composition.

10. A composition according to claims 1 - 7, **characterized in that** the amount of zinc oxide ranges from 0.5-10 wt. %, based on the weight of the overall composition.

11. A composition according to claims 1 - 7, **characterized in that** amount of additional components as referred to in claim 5 ranges from 10-50 wt. %, based on the weight of the overall composition.

12. A composition according to claims 1 - 11, **characterized in that** it is applied in the form of a liquid solution.

13. A composition according to claims 1 - 11, **characterized in that** it is applied in the form of an ointment.

14. Use of the composition according to claims 1-13 for the preparation of a substance for the treatment of wounds.

## Patentansprüche

1. Zusammensetzung, welche Honig als ein therapeutisch wirkendes Präparat zur Behandlung von Wunden und ferner Lanolin und/oder Lanolin-Derivate aufweist, **dadurch gekennzeichnet, dass** der Honig einen Peroxidanteil > 5 µg/g Honig Stunde aufweist, gemessen bei 21 °C..

2. Zusammensetzung nach Anspruch 1, **dadurch gekennzeichnet, dass** niedrig allergenes Lanolin verwendet wird.

3. Zusammensetzung nach Anspruch 1-2, **dadurch gekennzeichnet, dass** die Zusammensetzung ferner Zinkoxid aufweist.

4. Zusammensetzung nach Anspruch 3, **dadurch gekennzeichnet, dass** das Zinkoxid eine Partikelgröße < 0,1 µm aufweist.

5. Zusammensetzung nach Anspruch 1-4, **dadurch gekennzeichnet, dass** die Zusammensetzung eine oder mehrere zusätzliche Komponenten aus der Gruppe der Anti-Oxidantien, fully-trans-Vitamin A Säuren, vielfach ungesättigte Fettsäuren, n-Hexacosanol, Bis(Maltolato) Oxovanadium(IV), Aloe Vera, Ringelblumenöl, Ascorbyl Palmitat, Ascorbinsäure, Vitamin E, Verdickungsmittel, wie Carboxymethyl Cellulose, Polyethylenglycol und Stärke aufweist, welche sowohl modifiziert oder auch nicht modifiziert vorkommen können.

6. Zusammensetzung nach Anspruch 1-5, **dadurch gekennzeichnet, dass** der Honig einer Sterilisationsbehandlung unterzogen wurde, insbesondere eine Sterilisation mittels Gammastrahlung.

7. Zusammensetzung nach Anspruch 1-6, **dadurch gekennzeichnet, dass** der Honig frei von Schwermetallen, Pestiziden und Herbiziden ist.

8. Zusammensetzung nach Anspruch 1-7, **dadurch gekennzeichnet, dass** der Honiganteil zwischen 5-95 Gewichtsprozent bezogen auf das Gesamtgewicht der Zusammensetzung beträgt.

9. Zusammensetzung nach Anspruch 1-7, **dadurch gekennzeichnet, dass** der Lanolinanteil und/oder Lanolinderivatanteil zwischen 5-95 Gewichtsprozent bezogen auf das Gesamtgewicht der Zusammensetzung beträgt.

10. Zusammensetzung nach Anspruch 1-7, **dadurch gekennzeichnet, dass** der Zinkoxidanteil von zwischen 0,5-10 bezogen auf das Gesamtgewicht der Zusammensetzung beträgt.

11. Zusammensetzung nach Anspruch 1-7, **dadurch gekennzeichnet, dass** der Anteil der zusätzlichen Komponenten gemäß Anspruch 5 im Bereich zwischen 10-50 Gewichtsprozent bezogen auf das Gesamtgewicht der Zusammensetzung beträgt.

12. Zusammensetzung nach Anspruch 1-11, **dadurch gekennzeichnet, dass** dieser in Form von einer flüssigen Lösung angewendet wird.

13. Zusammensetzung nach Anspruch 1-11, **dadurch gekennzeichnet, dass** dieses in Form einer Salbe angewendet wird.

14. Verwendung einer Zusammensetzung nach Anspruch 1-13 für die Zubereitung einer Substanz zur Behandlung von Wunden.

## Revendications

1. Composition comprenant du miel comme composé thérapeutiquement actif pour le traitement de plaies, laquelle composition comprend en outre de la lanoline et/ou un dérivé de lanoline, **caractérisée en ce que** ledit miel a un nombre de peroxydes > 5 µg/g de miel par heure, mesuré à 21°C.

2. Composition selon la revendication 1, **caractérisée en ce que** la lanoline utilisée est du type faiblement allergénique.

3. Composition selon les revendications 1 et 2, **caractérisée en ce que** ladite composition comprend en outre de l'oxyde de zinc.

4. Composition selon la revendication 3, **caractérisée en ce que** l'oxyde de zinc utilisé présente une taille de particules < 0,1 µm.

5. Composition selon les revendications 1 à 4, **caractérisée en ce que** ladite composition comprend un ou plusieurs composants supplémentaires choisis dans le groupe des antioxydants, vitamine A acide tout trans, acides gras poly-insaturés, n-hexacosanol, bis(maltolato)oxovanadium(IV), aloe vera, huile de calendula, palmitate d'ascorbyle, acide ascorbique, vitamine E, épaississant, tel que la carboxyméthylcellulose, polyéthylène glycol et amidon, qui peuvent être modifiés ou non.

6. Composition selon les revendications 1 à 5, **caractérisée en ce que** ledit miel a été soumis à un traitement de stérilisation, dans une stérilisation particulière au moyen de rayons gamma.

7. Composition selon les revendications 1 à 6, **caractérisée en ce que** ledit miel est exempt de métaux lourds, de pesticides et d'herbicides.

8. Composition selon les revendications 1 à 7, **caractérisée en ce que** la quantité de miel varie de 5 à 95 % en poids, en se basant sur le poids de la composition globale.

9. Composition selon les revendications 1 à 7, **caractérisée en ce que** la quantité de lanoline et/ou de dérivé de lanoline varie de 5 à 95 % en poids, en se basant sur le poids de la composition globale.

10. Composition selon les revendications 1 à 7, **caractérisée en ce que** la quantité d'oxyde de zinc varie de 0,5 à 10 % en poids, en se basant sur le poids de la composition globale.

11. Composition selon les revendications 1 à 7, **caractérisée en ce que** la quantité de composants supplémentaires dont il est fait référence dans la revendication 5 varie de 10 à 50 % en poids, en se basant sur le poids de la composition globale.

12. Composition selon les revendications 1 à 11, **caractérisée en ce qu'**elle est appliquée sous la forme d'une solution liquide.

13. Compositions selon les revendications 1 à 11, **caractérisée en ce qu'**elle est appliquée sous la forme d'une pommade.

14. Utilisation de la composition selon les revendications 1 à 13 pour la préparation d'une substance destinée au traitement de plaies.
